(19) Europäisches Patentamt

European Patent Office

Office européen des brevets

(11) **EP 4 674 983 A1**

(12) **EUROPEAN PATENT APPLICATION**

(43) Date of publication:
**07.01.2026 Bulletin 2026/02**

(21) Application number: **24185803.4**

(22) Date of filing: **01.07.2024**

(51) International Patent Classification (IPC):
***C12Q 1/70*** *(2006.01)*

(52) Cooperative Patent Classification (CPC):
(C-Sets available)
**C12Q 1/702;** C12Q 1/6809; C12Q 2600/16   (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Ares Trading S.A.
1170 Aubonne (CH)**

(72) Inventors:
• **MAUTINO, Alessandro
10010 Colleretto Giacosa TO (IT)**

• **CUCURACHI, Marco
10010 Colleretto Giacosa TO (IT)**
• **CONTE, Daniele
10010 Colleretto Giacosa TO (IT)**
• **OLGIATI, Simone
10010 Colleretto Giacosa TO (IT)**
• **PRADUROUX, Alice
10010 Colleretto Giacosa TO (IT)**

(74) Representative: **Merck Serono S.A.
Intellectual Property
Terre Bonne Business Park
Building Z0
Route de Crassier 1
1262 Eysins (CH)**

(54) **METHOD FOR DETERMINING THE PRESENCE OF RETROVIRAL-LIKE PARTICLES**

(57)    Provided herein is a method for determining RetroViral Like Particles (RVLPs) using ddPCR (digital droplet PCR). The method enables screening of samples from a manufacturing process for biologic materials to be used as medicaments. As such the method can be used in batch release processes.

**(Cont. next page)**

EP 4 674 983 A1

(52) Cooperative Patent Classification (CPC): (Cont.)

C-Sets
C12Q 1/6809, C12Q 2531/113, C12Q 2563/159

## Description

### Field of the Invention

The present invention is in the filed of diagnostics in the manufacturing process for biologic materials. Such biologic materials may be used in medicaments for the treatment of mammalians, including medicaments for the treatment of humans. Provided herein is a method for determining RetroViral Like Particles (RVLPs) using ddPCR (digital droplet PCR). The method enables screening of samples from a manufacturing process for biologic materials to be used as medicaments. As such the method can be used in batch release processes.

### Background

In the manufacturing of medicaments, particularly those that use biologic methods such in the production of biologic material for human (or mammalian) medicaments, screening for the presence or absence of RetroViral Like Particles (RLVPs) is an important aspect for determining the suitable for use as a medicament. The current method uses "Virus Particle Detection and Quantification by Ultracentrifugation and Thin Section Electron Microscopy" method (hereinafter "TEM method"). The TEM method remains the reference method for commercial products or products under clinical trials.

The Chinese Hamster Ovary (CHO) cells used to produce biopharmaceutical proteins are known to contain type-C endogenous retrovirus (ERV) sequences in their genome and to release retroviral-like particles (RVLP). Although RVLPs are shown to be non-infectious and not associated with any diseases in humans, their presence raises safety and regulatory concerns. For instance, there is a risk that these RVLPs lead to a possible hamster to human transmission. Moreover, these RVLPs can also interfere with the detection of other adventitious agents.

The quantitative estimation of RVLPs in unprocessed bulk harvest material is therefore important to assess the adequacy of virus removal/inactivation determined by the viral clearance studies for the entire manufacturing process. The entire purification process should be able to eliminate substantially more virus than is estimated to be present in a single-dose equivalent of unprocessed bulk.

Currently, the analytical method applied for the RVLPs detection in unprocessed bulk harvest samples is the TEM method "Virus Particle Detection and Quantification by Ultracentrifugation and Thin Section Electron Microscopy". The main principles of this method are summarized as follows:

After clarification and ultra-centrifugation, the resultant pellet is re-suspended and mixed with agar. The sample/agar matrix is then fixed, stained, dehydrated, infiltrated to resin and polymerized. Ultrathin sections are cut with an ultra-microtome and mounted on electron microscope grids. After staining, the sections are examined by TEM (Transmission Electron Microscopy) in such a way that no grid square is scored more than once.

The grid squares are examined for the presence of viruses, virus-like particles and other extraneous agents. A minimum of ten grid squares are examined and a count performed. In the event no viruses or virus-like particles are observed, the number of grid squares required to achieve a theoretical detection limit of $1.0 \times 10^5$ VLPs/ml of sample are examined.

It has been observed that there can be a marked variability in the results, even for the same samples. Accordingly, there is a need for an alternative reliable and accurate method for determining the presence or absence of RLVPs.

### Summary of the Invention

The method of the present invention is a method based on droplet digital PCR, "Retro-Virus Like Particles by droplet digital PCR (ddPCR) in Unprocessed Bulk Harvest Sample" for RVLP detection and has a better robustness, higher sensitivity and higher reproducibility.

The method of the present invention can be performed as follows: The sample is treated with DNase and RNase to eliminate most of the genomic DNA and RNA of circulating RVLP. This step allows to quantify only the RNA extracted from intact RVLP particles, each containing two copies of RNA. The RNA from intact RVLPs is then extracted (in 3 different replicates) using an automatic extraction system, amplified, and quantified using ddPCR system.

The droplet digital PCR (ddPCR) technology allows to determine the presence of target nucleic acids in biological samples and performs an absolute quantification. The absolute quantification of the nucleic acid molecules is possible thanks to a system distributing the sample into small droplets of water-oil emulsion one nanoliter in size.

After the sample is randomly distributed in the individual droplets in each of these, a PCR or RT-PCR reaction is performed with a fluorescent probe. Following the PCR reaction, the droplets are analyzed individually by measuring the fluorescence signal emitted by the probe. Based on the number of droplets that are positive or negative, and using the subsequent statistical calculations based on the Poisson distribution, the absolute quantity of nucleic acids present in the sample is obtained.

The method for determining RVLPs of the present invention includes that the sample is analyzed in single by ddPCR using two different amplification Mixes: one to amplify both RNA and DNA, which contains the Retro Transcription enzyme, and the other to amplify DNA only, which does not contain the Retro transcription enzyme.

Sample amplification with the two different Master Mixes allows the quantification of RVLP particles considering only the number of extracted RNA copies, thus excluding the number of copies derived from the genomic DNA still present in the extract. To calculate the Retrovirus-Like Particles in the sample, the value of copies' number obtained in each replicate of the sample, amplified with the RT-ddPCR Master Mix, is subtracted from the value of copies' number in each replicate of the sample amplified with the NO-RTddPCR Master Mix.

The final value of the number of RVLP particles present in the sample is given as the average of the RVLP/mL obtained in each replicate tested.

In one embodiment of the present invention for the method for determining RVLPs provides a method for determining RetroViral-Like Particles (RVLPs) in a sample, the method comprising

   a. Obtaining a biologic sample from a manufacturing process for the production of a biologic composition,

   b. Treating the biologic sample with both DNase and RNase to remove host cell genome and circulating RNA,

   c. Extracting nucleic acid material from the treated biologic sample of step b),

   d. Preparing two reaction mixtures, a first reaction mixture (RT Mix) for both DNA and RNA amplification and a second reaction mixture (NO-RT Mix) for DNA amplification,

   e. Adding an amount of the extracted nucleic acid materials from step c) to each of the two reaction mixtures of step d),

   f. Applying PCR amplification to each of the reaction mixtures of step e), and

   g. Determine the presence or absence of retrovirus materials by subtracting the results of the first and second part of the amplification of the extracted nucleic acid materials.

In a further embodiment of the method of the present invention, the first reaction mixture (RT-Mix) comprises reverse transcriptase and the second reaction mixture (No-RT Mix) does not comprise reverse transcriptase.

**Detailed Description**

The method of the present invention provides a robust, predictable and highly accurate method for the determination of RVLPs in a sample from a manufacturing process to prepare a biologic material that can be used for the use in a medicament for the treatment of humans/mammalians.

In one embodiment of the present invention for the method for determining RVLPs provides a method for determining RetroViral-Like Particles (RVLPs) in a sample, the method comprising

   a. Obtaining a biologic sample from a manufacturing process for the production of a biologic composition,

   b. Treating the biologic sample with both DNase and RNase to remove host cell genome and circulating RNA,

   c. Extracting nucleic acid material from the treated biologic sample of step b),

   d. Preparing two reaction mixtures, a first reaction mixture (RT Mix) for both DNA and RNA amplification and a second reaction mixture (NO-RT Mix) for DNA amplification,

   e. Adding an amount of the extracted nucleic acid materials from step c) to each of the two reaction mixtures of step d),

   f. Applying PCR amplification to each of the reaction mixtures of step e), and

   g. Determine the presence or absence of retrovirus materials by subtracting the results of the first and second part of the amplification of the extracted nucleic acid materials.

This method of the present invention better detects the presence of RVLPs than the current TEM methods. The method provides an increased accuracy in respect to TEM method due to the ability of the ddPCR method to discriminate RVLP from the empty particles with the detection of RNA component of RVLP. Moreover, the present invention provides a method with increased reproducibility in respect to TEM method observing a reduced result variability when the same batch of the same product is tested (lower than 25%). In addition, only a low sample volume is needed for testing (1 ml) (instead of 20 ml needed for TEM analysis). Also the method of the present invention provides decreased timelines for results availability (lead time of 14 days, instead of up to 80 days needed for TEM), thus better fitting with the new DS development timelines. Finally, the method of the present invention provides a better detection limit ($1.4 \times 10^4$ RVLPs/ml, instead of $1.0 \times 10^5$ RVLPs/ml for TEM). Therefore, the method of the present invention satisfies a need for having a faster method providing a better accuracy and sensitivity.

Table 1: Summary Table of the Benefits & Risks for ddPCR and TEM methods

| Method | Benefits | Risk |
|---|---|---|
| Retrovirus-Like Particles by droplet digital PCR (ddPCR) in Bulk Harvest Samples | • Good limit of quantification = $1.4 \times 10^4$ VLPs/ml<br>• Increase of results accuracy<br>• Low variability in results<br>• Reduced sample volume needed for the analysis (1ml)<br>• Reduced lead time for results availability (14 days) | |
| Virus Particle Detection and Quantification by Ultracentrifugation and Thin Section Electron Microscopy | • Limit of Quantification = $1.0 \times 10^5$ VLPs/ml, superior to other sectioning techniques where homogeneity of pellets is difficult to achieve | High variability in results: up to 3 log difference observed on the same batch of the same product<br>• High lead time for results availability (up to 80 days)<br>• Identification of RVLPs can prove difficult in sections under analysis |

Examples

Unprocessed Bulk Harvest of CTLA4_aOX (hereinafter reported as COX) and MSLN_CD137 (hereinafter reported as MN7) were tested. In addition, genomic extract from CHO-K1 cells containing type-C endogenous retrovirus (ERV) target sequence was used to test specificity, accuracy and method LOQ as a certified RNA standard was not available.

A panel of positive and negative controls were used to ensure the validity of the results.

• Linearity: Linearity was assessed as the ability of the method to quantify RVLP by ddPCR in 5 serial dilutions of Unprocessed bulk harvest samples (COX and MN7). The linearity of each sample was verified by evaluating the average % recovery of RVLP/ml obtained in the three extractions for each dilution in comparison to the first dilution point.

• Range: the range of the samples was defined as the dilution range in which linearity has been demonstrated.

• Specificity: the specificity of the method to amplify the endogenous retro-virus (ERV) target region in different types of CHO hosts (CHO-S, CHO-K1 and CHO-DUKX B11) and not in other host cells tested (NS0-LD hybridoma cells) was verified. In addition, it was also assessed the specificity of the primer and probe set to detect RVLPs and no other virus species (MVM).

• Accuracy: the accuracy in quantifying the sequence of the target gene for RVLP was verified by analysing three replicates for three different concentrations of genomic CHO-K1 extract and by valuating the recovery percentage of the endogenous retrovirus (ERV) copies/μl obtained in comparison to the expected value of RVLP copies/μl (defined in the specificity test).

• Precision: Repeatability was extrapolated from the linearity tests. CV% of RVLP/ml values obtained in the three linearity replicates for the initial dilution of the COX bulk harvest sample was calculated. To assess intermediate precision the same analysis was repeated by a second operator. The RVLP/mL values obtained were evaluated together with those obtained by the first operator in the linearity tests and the CV% of RVLP/ml was calculated on the replicates analysed by the two analysts.

- Limit of Quantification (LOQ): the LOQ of the method was assessed as the number of RVLP copies/ml that the ddPCR method is able to detect and quantify. The LOQ was verified by analysing 5 replicates of five serial dilutions of genomic CHO-K1 extract and by evaluating the CV%. The value of RVLP copies detected in the lowest dilution which showed a CV% < 25% was used to calculate the LOQ using the following formula:

$$RVLP\ (particles/mL) = x/2 * 100 * 1.5 * 5 * Dilution\ Factor/2$$

Where:

- x (copies/20 $\mu$L) is the difference between the number of copies 20 $\mu$L obtained with the RT-ddPCR Master Mix and the number of copies 20 $\mu$L obtained with the NO-RT-ddPCR Master Mix.

- 2 is the division factor considering that 2 $\mu$L of extracted is loaded into the reaction.

- 100 is the multiplication factor considering the extract volume.

- 1.5 is the multiplication factor considering that the sample (200 $\mu$L) is diluted before extraction up to a volume of 300 $\mu$L.

- 5 is the multiplication factor to report the number of copes in mL, considering that 200 $\mu$L sample is extracted.

- Dilution Factor is the dilution factor of the sample in question.

- 2 is the division factor considering that each RVLP particle contains two copies of RNA.

The above formula was also used for the calculation of LOQ related to the samples COX and MN7, using as a dilution factor the results obtained in the test of the Linearity and Range of the samples.
• Robustness: the robustness was assessed by verifying the performance of the analytical method when deliberate changes to the values set for the critical steps are applied. Changes were introduced on the duration of DNAse-RNAse, DNAse and inactivation step incubations.

Table 2: Comparison of Validation Results between Current (TEM) vs ddPCR method for RVLP detection

| Validation parameter | TEM (Current method) | | ddPCR (Proposed method) | | conclusion |
|---|---|---|---|---|---|
| | Validation acceptance criteria | Validation results | Vaildation accaptance criteria | validation results | |
| Linearity | Average titre for each sample dilutions tested in three replicates within 1 $\log_{10}$ of the expected titre. | TEM method is linear for each dilution tested (average titre within 1$\log_{10}$ of their average). | Average recovery % of the RVLPs quantity between 50% and 150% in at least two out of five consecutive sample dilutions, in comparison to the first dilution point | COX sample lineanty verified in dilutions from 1:2 to 1:32

MN7 sample: linearity verified in dilutions from 1:1 to 1:16 | ddPCR method is linear for the sample ranges tested

COX: dilutions from 1:2 to 1:32

MN7: dilutions from 1:1 to 1:16 |
| Range | N.A. | N.A. | Range of dilution at which samples shows linearity could be tested. | COX sample: dilutions from 1:2 to 1:32

MN7 sample, dilutions from 1:1 to 1:16 | ddPCR sample ranges:
COX: dilutions from 1:2 to 1:32

MN7: dilutions from :1 to 1:16 |

(continued)

| Validation parameter | TEM (Current method) | | ddPCR (Proposed method) | | conclusion |
|---|---|---|---|---|---|
| | Validation acceptance criteria | Validation results | Vaildation accaptance criteria | validation results | |
| Specificity | Specificity considered in terms of the suitability of the system to allow detection and quantification of RVLPs: - virus particles of in- house reference material tested should be readily distinguishable and countable. - bulk harvest sample known to contain RVLPs should confirm that RVLPs are distinguishable | The method is considered suitable for detecting and quantifying RVLP as particles demonstrated the structural characteristics expected for RVLPs and were clearly distinguished by direct counting (for both reference material and bulk harvest samples). | CHO-S, CHO-K1 and CHO-DUKX: RVLPs copies/$\mu$l > 100 and percent variation of 3 replicates for each cell line $\leq$ 25%. NS0-LD and MYM samples RVLPs copies/$\mu$l < 5. | Specificity confirmed. CHO-S, CHO-K1, CHO-DUKX: - RVLPs copies/$\mu$l > 100 - % variation of 3 replicates for each cell line $\leq$ 3%. NS0-LD and MVM samples: RVLPs copies/$\mu$l undetermined (< 5) | ddPCR method is specific for amplifying the RVLPs target in CHO host cell lines In addition, ddPCR assay is specific for quantifying only RVLP in Unprocessed Bulk Harvest samples |
| Accuracy | Average for all replicates of the undiluted reference stock tested within 1 $\log_{10}$ of the reference titre ($7.7 \times 10^8$ RVLP/ml). | Accuracy confirmed Average for all replicates of the sample within 1$\log_{10}$ (results obtained from 6.0 $\times 10^8$ to 9.9 $\times 10^8$ RVLP/ml) | Average % recovery of the RVLPs copies/$\mu$l obtained for three replicates of three concentrations of genomic CHO-K1 DNA, between 75 % and 125% in comparison to the expected values | Accuracy confirmed. Average % recovery of the RVLPs copies/$\mu$l for the three concentrations of genomic CHO-K1 DNA between 96% and 106%. | ddPCR method is accurate at quantifying RVLP. |
| Precision | Repeatability and Intermediate Precision: all individual titres tested within 1$\log_{10}$ of their average | TEM method is precise both in terms of repeatability and intermediate precision (all individual titres tested were within 1$\log_{10}$ of their average) | Repeatability: CV% of RVLP/ml obtained $\leq$ 25%. Intermediate Precision: CV% of RVLP/ml obtained from two different operators $\leq$ 25%. | Repeatability CV% = 5% Intermediate precision: CV% = 12% | ddPCR method is precise both in terms of Repeatability and Intermediate Precision |

(continued)

| Validation parameter | TEM (Current method) | | ddPCR (Proposed method) | | conclusion |
|---|---|---|---|---|---|
| | Validation acceptance criteria | Validation results | Vaildation accaptance criteria | validation results | |
| Limit Of Quantification | LOQ determined theoretically based on the assumption of random distribution (Poisson) for counts and on the volume (or surface area) tested | TEM method LOQ is $1.0 \times 10^5$ RVLP/ml | LOQ calculated basing on the number of RVLPs copies/20µl obtained in the lowest genomic CHO-K1 extract dilution with a CV% $\leq$ 25% | 74.1 RVLPs copies/20µl were obtained in the lowest dilution showing CV% $\leq$ 25%. The copies value corresponds to $1.35 \times 10^4$ RVLP/ml. | ddPCR method LOQ is $1.39 \times 10^4$ RVLP/ml, improved respect to the TEM LoQ |
| Robustness | N.A. | N.A. | Data obtained by testing critical parameters (duration of DNAse-RNAse treatment, DNAse and sample inactivation incubations): global variance: Prob>F (0.05) | Global vanance not significant (Prob = 0.2856). | ddPCR method was demonstrated to be robust despite changes in the duration of DNAse-RNAse, DNAse and sample inactivation incubations |

In the frame of the development and validation of ddPCR method for RVLPs detection and quantification, Unprocessed Bulk Harvest samples deriving from different manufacturing processes were tested with both TEM and ddPCR methods.

In particular, Unprocessed bulk harvest samples collected from perfusion bioreactors at different Working Days (WD), from the steady state to the end of the production phase, were tested from MSLN_CD137 (MN7), CTLA4_aOX40L (COX) and Anti-GD2 ADC processes production, at different scales (50 L, 200L or 2000 L).

The results obtained are detailed in the Tables reported in following paragraph.

Table 3: Results for MSLN_CD137 (MN7) GMP run 200 L

| Sample batch | TEM results (RVLP/ml) | ddPCR results (RVLP/ml) |
|---|---|---|
| BMCRP80003 WD08BIO | $2.1 \times 10^5$ | / |
| BMCRP80003 WD09BIO | / | $3.4 \times 10^5$ |
| BMCRP80003 WD10BIO | $2.9 \times 10^5$ | / |
| BMCRP80003 WD12BIO | / | $2.7 \times 10^5$ |
| BMCRP80003 WD13BIO | $4.4 \times 105$ | / |
| BMCRP80003 WD16BIO | $1.0 \times 10^5$ | / |
| BMCRP80003 WD18BIO | / | $1.9 \times 10^5$ |
| BMCRP80003 WD20BIO | $3.4 \times 10^5$ | / |
| BMCRP80003 WD22BIO | / | $3.2 \times 10^5$ |
| BMCRP80003 WD24BIO | $6.0 \times 10^5$ | / |

Table 4: Results for CTLA4_aOX40L (COX) Fine-tuning run, GLP run and GMP run

| Run | Sample Batch | TEM results (RVLP/ml) | ddPCR results (RVLP/ml) |
|---|---|---|---|
| Fine-tuning (50L) | CTLA4-aOX40L A COX-041-WD11 | $4.6 \times 10^6$ | $2.30 \times 10^6$ |
| | CTLA4-aOX40L A COX-041-WD16 | $4.6 \times 10^6$ | $2.38 \times 10^6$ |
| | CTLA4-aOX40L A COX-041-WD22 | No VLPs observed with detection limit of $1 \times 10^5$ | $5.45 \times 10^5$ |
| | CTLA4-aOX40L A COX-041-WD28 | $6.0 \times 10^6$ | $1.43 \times 10^6$ |
| | CTLA4-aOX40L A COX-041-WD35 | $9.0 \times 10^6$ | $3.11 \times 10^6$ |
| | CTLA4-aOX40L A COX-041-WD38 | $7.5 \times 10^6$ | $2.99 \times 10^6$ |
| GLP run (200 L) | BCXRP78001_BIO_WD09 | $2.4 \times 10^6$ | $1.09 \times 10^6$ |
| | BCXRP78001_BIO_WD14 | $6.3 \times 10^6$ | $1.15 \times 10^6$ |
| | BCXRP78001_BIO_WD16 | $6.8 \times 10^6$ | $1.51 \times 10^6$ |
| | BCXRP78001_BIO_WD19 | $1.0 \times 10^7$ | $1.81 \times 10^6$ |
| | BCXRP78001_BIO_WD22 | $2.3 \times 10^7$ | $2.14 \times 10^6$ |
| | BCXRP78001_BIO_WD25 | $2.6 \times 10^7$ | $2.62 \times 10^6$ |
| GMP run (2000 L) | BCXRP82001_crWD09 | $1.5 \times 10^8$ | $2.22 \times 10^6$ |
| | BCXRP82001_crWD12 | $6.0 \times 10^7$ | $1.57 \times 10^6$ |
| | BCXRP82001_crWD16 | $1.3 \times 10^8$ | $3.70 \times 10^6$ |
| | BCXRP82001_crWD18 | $4.8 \times 10^7$ | $7.09 \times 10^6$ |
| | BCXRP82001_crWD20 | $4.0 \times 10^7$ | $1.69 \times 10^7$ |
| | BCXRP82001_crWD22 | $4.6 \times 10^7$ | $7.27 \times 10^6$ |

Table 5: Results for Anti-GD2 GLP run

| Sample batch (GLP 200 L run) | TEM results (RVLP/ml) | ddPCR results (RVLP/ml) |
|---|---|---|
| BGDRP78002_CrWD08 | No VLPs observed with detection limit of $1 \times 10^5$ | $1.57 \times 10^5$ |
| BGDRP78002_CrWD10 | No VLPs observed with detection limit of $1 \times 10^5$ | $4.23 \times 10^5$ |
| BGDRP78002_CrWD12 | No VLPs observed with detection limit of $1 \times 10^5$ | $4.70 \times 10^5$ |
| BGDRP78002_CrWD15 | $7.5 \times 10^6$ | $1.30 \times 10^6$ |
| BGDRP78002_CrWD17 | $2.7 \times 10^7$ | $1.57 \times 10^6$ |
| BGDRP78002_CrWD19 | $5.5 \times 10^7$ | $1.74 \times 10^6$ |

As shown in the reported tables, the results obtained with the ddPCR method in terms of RVLP/ml are lower than the ones obtained with TEM analysis. Indeed, as the method is focusing on RNA component of RVLP, the ddPCR method is able to discriminate the RVLPs from the empty particles which are, on the other hand, not distinguishable by TEM, providing a more reliable and representative quantification of RVLP. In addition, the ddPCR method was demonstrated to be more precise and accurate as no overestimation of RVLP content is shown.

With ddPCR method, the same trend in RVLPs quantification was observed for each molecule and run tested. Indeed, the highest results in terms of RVLP quantification were observed in the last Working Day (WD) (MN7 GMP, COX GLP and Anti-GD2 GLP runs) and second last WD (COX fine-tuning and COX GMP runs) for all the processes tested.

With TEM method, the same trend in RVLPs quantification was observed for MN7 GMP, COX fine-tuning, COX GLP and Anti-GD2 GLP runs with the highest results in terms of RVLPs in the last WD (MN7 GMP and COX fine-tuning runs) and second last WD (COX GLP and Anti- GD2 GLP runs).

However, a different trend was observed for COX GMP run as the highest results in terms of RVLPs were observed in the first WDs. These results are not in line with the ones obtained by ddPCR analysis and by TEM method in the other process runs.

These results are not considered representative for the following reasons:

- Protein retention observed in the crude harvest by using an Alternative Tangential Flow (ATF) as cell separation system could be used as a potential surrogate for RVLP retention. For COX GMP run, as the protein retention increased significantly along the culture duration ($\approx$ 0% at WD9 versus $\approx$43% at WD22), an increased RVLP retention would be expected at the end of the culture duration, resulting in higher RVLP levels in the lasts WD, as observed for all other runs and molecules tested by ddPCR and TEM (MN7 GMP, COX fine-tuning and GLP, and Anti-GD2 GLP runs)

- As described, the ddPCR method is more robust, precise, providing a more reliable and accurate quantification of RVLP than the TEM method as no overestimation of RVLP content is shown.

- The TEM method is highly variable, based on the results obtained from prior knowledge data, a difference in terms of RVLPs retention up to 3 log was observed by analyzing the same batch of the same product, justifying the high variability of the method.

For all the reasons reported above, results obtained with TEM method for COX GMP run for WD09 and WD16 samples can be considered as outliers. In conclusion, ddPCR is a more appropriate method to quantify RVLP for perfusion processes where the trend of RVLP production and variations along the culture duration can be established with more accuracy and precision.

The retroviral-like particle count in the Unprocessed bulk harvest is needed to assess the potential viral load per dose of drug product, representing the safety margin of the process. The RVLP testing strategy needs to be adapted depending on the type of culture (fed-batch mode versus perfusion mode). For fed-batch processes, the RVLP testing of the Unprocessed bulk harvest is done at the end of the production phase before the clarification step, where the RVLP level in the bioreactor is the highest.

For perfusion processes, the cell culture duration might be extended with a continuous forward processing of harvested cell culture material. For this reason, the periodicity of the testing and the representativeness of the samples should be justified, due to the potential for unprocessed bulk to be continually harvested from the production reactor during processing and for the potential for the virus like particles to variate along the cell culture span. There is therefore a need to increase the frequency of sampling. It was assessed that a number of 6 samples, sampled at 6 different Working Days (WD) during the production phase (including the last WD of the culture), from the steady state to the end of the culture, would be suitable to get a trend of RVLP production along the perfusion culture.

In case of change of the cell separation technology (i.e., ATF) during the culture, a testing needs to be performed the WD before and after the change. Indeed, the potential sieving of RVLP due to the progressive filter fouling would be worst-case in terms of RVLP retention and safety margin estimation.

Thus, the present invention provides a high sensitivity and accurate droplet digital PCR (ddPCR) method for detecting and quantifying Retrovirus-like particles (RVLPs) in Unprocessed Bulk Harvest samples as alternative to TEM. This method is based on ddPCR technology, allowing an absolute quantification of RVLPs, focusing on RNA component.

The system was shown to provide highly precise and reproducible quantification of RVLPs starting from a low sample volume (1 ml) and good Limit Of Quantification ($1.4 \times 10^4$ RVLPs/ml).

A comparability exercise performed on three molecules (COX, MN7 and Anti-GD2) for 5 different runs at different scales (50L, 200L, 2000L, ref. paragraph 7) showed comparable RVLP results quantified by both methods. The same trend in RVLPs quantification was observed along the perfusion processes for each run, with the highest results observed in the lasts WD, except for COX GMP run results obtained by TEM.

**Claims**

1. A method for determining RetroViral-Like Particles (RVLPs) in a sample, the method comprising

    a. Obtaining a biologic sample from a manufacturing process for the production of a biologic composition,
    b. Treating the biologic sample with both DNase and RNase to remove host cell genome and circulating RNA,
    c. Extracting nucleic acid material from the treated biologic sample of step b),
    d. Preparing two reaction mixtures, a first reaction mixture (RT Mix) for both DNA and RNA amplification and a second reaction mixture (NO-RT Mix) for DNA amplification,
    e. Adding an amount of the extracted nucleic acid materials from step c) to each of the two reaction mixtures of step d),

f. Applying PCR amplification to each of the reaction mixtures of step e), and

g. Determine the presence or absence of retrovirus materials by subtracting the results of the first and second part of the amplification of the extracted nucleic acid materials.

2. The method of claim 1, wherein the PCR amplification is a droplet digital PCR (ddPCR) amplification.

3. The method of any of the preceding claims, further treating the material from step b) with DNase only before step c).

4. The method of any of the preceding claims, wherein the host cell is a Chinese Hamster Ovary (CHO) cell.

5. The method of any of the preceding claims, wherein the first reaction mixture (RT-Mix) comprises reverse transcriptase and the second reaction mixture (No-RT Mix) does not comprise reverse transcriptase.

6. The method of claim 5, wherein the preparation of the two mixtures from step d) is performed with:

for the first reaction mixture (RT Mix):

- 5 $\mu$l /well of SuperMix
- 2 $\mu$l/well of Reverse Transcriptase
- 1 $\mu$l/well of 300 mM DTT
- 0.225 $\mu$l/well of RVLP Forward Primer 80 $\mu$M (sequence CCTCCTCCAGACTCTTG)
- 0.225 $\mu$l/well of RVLP Reverse Primer 80 $\mu$M (sequence ATACGGGCTTCCGTTAG)
- 0.5 $\mu$l/well of RVLP Probe 10 $\mu$M (sequence CCAAGAAGGCCCAGATATGTCA)
- 8.1 $\mu$l/well of Water for Molecular Biology

and for the second reaction mixture (NO-RT Mix):

- 5 $\mu$l /well of SuperMix
- 1 $\mu$l/well of 300 mM DTT
- 0.225 $\mu$l/well of RVLP Forward Primer 80 $\mu$M (sequence CCTCCTCCAGACTCTTG)
- 0.225 $\mu$l/well of RVLP Reverse Primer 80 $\mu$M (sequence ATACGGGCTTCCGTTAG)
- 0.5 $\mu$l/well of RVLP Probe 10 $\mu$M (sequence CCAAGAAGGCCCAGATATGTCA)
- 11.5 $\mu$l/well of Water for Molecular Biology

Europäisches
Patentamt

European
Patent Office

Office européen
des brevets

## EUROPEAN SEARCH REPORT

**Application Number**

EP 24 18 5803

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HUSSAIN MUSADDEQ ET AL: "A direct RT qPCR method for quantification of retrovirus-like particles in biopharmaceutical production with CHO cells", JOURNAL OF PHARMACEUTICAL AND BIOMEDICAL ANALYSIS, vol. 189, 1 September 2020 (2020-09-01), page 113472, XP093231908, AMSTERDAM, NL ISSN: 0731-7085, DOI: 10.1016/j.jpba.2020.113472 * the whole document * ----- | 1-5 | INV. C12Q1/70 |
| Y | LIMING SHI ET AL: "Real time quantitative PCR as a method to evaluate xenotropic murine leukemia virus removal during pharmaceutical protein purification", BIOTECHNOLOGY AND BIOENGINEERING, JOHN WILEY, HOBOKEN, USA, vol. 87, no. 7, 24 August 2004 (2004-08-24), pages 884-896, XP071151537, ISSN: 0006-3592, DOI: 10.1002/BIT.20198 * the whole document * ----- | 1-5 | **TECHNICAL FIELDS SEARCHED (IPC)**<br><br>C12Q |
| Y | MIN ZHANG ET AL: "Quality by design approach for viral clearance by protein a chromatography", BIOTECHNOLOGY AND BIOENGINEERING, vol. 111, no. 1, 1 January 2014 (2014-01-01), pages 95-103, XP055233367, Hoboken, USA ISSN: 0006-3592, DOI: 10.1002/bit.24999 * the whole document * ----- -/-- | 1-5 | |

The present search report has been drawn up for all claims

| Place of search | Date of completion of the search | Examiner |
|---|---|---|
| The Hague | 9 December 2024 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.....................................................................................

& : member of the same patent family, corresponding document

EPO FORM 1503 03.82 (P04C01)

**page 1 of 4**

Europäisches
Patentamt

European
Patent Office

Office européen
des brevets

## EUROPEAN SEARCH REPORT

**Application Number**

EP 24 18 5803

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DE WIT CHRISTINA ET AL: "Real-time Quantitative PCR for Retrovirus-like Particle Quantification in CHO Cell Culture", BIOLOGICALS, vol. 28, no. 3, 1 September 2000 (2000-09-01), pages 137-148, XP093231907, GB ISSN: 1045-1056, DOI: 10.1006/biol.2000.0250 * the whole document * ----- | 1-5 | |
| Y | US 2022/081726 A1 (RAMOS KENNETH [US] ET AL) 17 March 2022 (2022-03-17) * example 1 * * page 112 * ----- | 1-5 | |
| Y | C. A. KAHL ET AL: "Human Immunodeficiency Virus Type 1-Derived Lentivirus Vectors Pseudotyped with Envelope Glycoproteins Derived from Ross River Virus and Semliki Forest Virus", JOURNAL OF VIROLOGY, vol. 78, no. 3, 1 February 2004 (2004-02-01), pages 1421-1430, XP055199255, ISSN: 0022-538X, DOI: 10.1128/JVI.78.3.1421-1430.2004 * the whole document * ----- -/-- | 1-5 | TECHNICAL FIELDS SEARCHED (IPC) |

The present search report has been drawn up for all claims

| Place of search | Date of completion of the search | Examiner |
|---|---|---|
| The Hague | 9 December 2024 | Bruma, Anja |

EPO FORM 1503 03.82 (P04C01)

Europäisches
Patentamt

European
Patent Office

Office européen
des brevets

**EUROPEAN SEARCH REPORT**

Application Number

EP 24 18 5803

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KURT BRORSON ET AL: "Use of a Quantitative Product-Enhanced Reverse Transcriptase Assay to Monitor Retrovirus Levels in mAb Cell-Culture and Downstream Processing", BIOTECHNOLOGY PROGRESS, AMERICAN CHEMICAL SOCIETY, HOBOKEN, USA, vol. 17, no. 1, 5 September 2008 (2008-09-05), pages 188-196, XP072292944, ISSN: 8756-7938, DOI: 10.1021/BP000153Q * the whole document * ----- | 1-5 | |
| Y | SYDNEY B. BLATTMAN ET AL: "Prokaryotic Single-Cell RNA Sequencing by In SituCombinatorial Indexing", BIORXIV, 6 December 2019 (2019-12-06), XP055673410, DOI: 10.1101/866244 Retrieved from the Internet: URL:https://www.biorxiv.org/content/10.110 1/866244v1.full.pdf> * page 10, paragraph 1-2 * ----- | 1-5 | |
| Y | SIKORA ANNE-SOPHIE ET AL: "Regulation of the Expression of Heparan Sulfate 3- O -Sulfotransferase 3B (HS3ST3B) by Inflammatory Stimuli in Human Monocytes", JOURNAL OF CELLULAR BIOCHEMISTRY, vol. 117, no. 7, 1 July 2016 (2016-07-01), pages 1529-1542, XP093133497, Hoboken, USA ISSN: 0730-2312, DOI: 10.1002/jcb.25444 * page 1531, column 1, last paragraph - column 2, paragraph first * ----- -/-- | 1-5 | TECHNICAL FIELDS SEARCHED (IPC) |

The present search report has been drawn up for all claims

| Place of search | Date of completion of the search | Examiner |
|---|---|---|
| The Hague | 9 December 2024 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................................

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 24 18 5803

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2019/338262 A1 (LEONARD JOSHUA N [US] ET AL) 7 November 2019 (2019-11-07) * paragraph [0146] * | 1-5 | |
| A | LOVATT A ET AL: "HIGH THROUGHPUT DETECTION OF RETROVIRUS-ASSOCIATED REVERSE TRANSCRIPTASE USING AN IMPROVED FLUORESCENT PRODUCT ENHANCED REVERSE TRANSCRIPTASE ASSAY AND ITS COMPARISON TO CONVENTIONAL DETECTION METHODS", JOURNAL OF VIROLOGICAL METHODS, ELSEVIER BV, NL, vol. 82, 1 January 1999 (1999-01-01), pages 185-200, XP002940035, ISSN: 0166-0934, DOI: 10.1016/S0166-0934(99)00111-1 * the whole document * | 1-6 | |

TECHNICAL FIELDS
SEARCHED (IPC)

The present search report has been drawn up for all claims

| Place of search | Date of completion of the search | Examiner |
|---|---|---|
| The Hague | 9 December 2024 | Bruma, Anja |

EPO FORM 1503 03.82 (P04C01)

page 4 of 4

## ANNEX TO THE EUROPEAN SEARCH REPORT
## ON EUROPEAN PATENT APPLICATION NO.

EP 24 18 5803

This annex lists the patent family members relating to the patent documents cited in the above-mentioned European search report.
The members are as contained in the European Patent Office EDP file on
The European Patent Office is in no way liable for these particulars which are merely given for the purpose of information.

09-12-2024

| Patent document cited in search report | Publication date | Patent family member(s) | Publication date |
|---|---|---|---|
| US 2022081726 A1 | 17-03-2022 | US 2022081726 A1<br>WO 2020142436 A1 | 17-03-2022<br>09-07-2020 |
| US 2019338262 A1 | 07-11-2019 | US 2019338262 A1<br>WO 2018081039 A1 | 07-11-2019<br>03-05-2018 |

EPO FORM P0459

For more details about this annex : see Official Journal of the European Patent Office, No. 12/82